# EUROPEAN PATENT SPECIFICATION

(11) **EP 2 178 818 B1**
(45) Date of publication and mention of the grant of the patent: **09.09.2020**
(21) Application number: 08848777.2
(22) Date of filing: 21.07.2008
(51) Int. Cl.: C07C 50/12, A61K 31/122, A61P 7/00, A61P 5/48, A61P 17/00

(54) **TREATMENTS USING VITAMIN K ANALOGUES AND DERIVATIVES**
BEHANDLUNGEN MIT VITAMIN-K-ANALOGEN UND -DERIVATEN
TRAITEMENTS UTILISANT DES ANALOGUES ET DERIVES DE LA VITAMINE K

(30) Priority: 24.07.2007 IN MU14192007
(43) Date of publication of application: 28.04.2010
(73) Proprietor: Viridis Biopharma Pvt Ltd., Maharashtra (IN)
(72) Inventor: VAIDYA, Ashok, B., Maharashtra (IN); MEHTA, Dilip, S., Maharashtra (IN); DE SOUZA, Anselm, Maharashtra (IN); VAIDYA, Rama, A., Maharashtra (IN)
(74) Representative: Forrest, Stuart
(86) International application number: PCT/IN2008/000465
(87) International publication number: WO 2009/063485

(56) References cited:
- WO-A1-97/39746
- WO-A1-2004/064798
- DE-U1-202004 013 521
- US-A- 5 510 391
- US-A- 5 525 478
- US-A1- 2002 025 983
- US-A1- 2003 190 369
- US-A1- 2004 167 207
- US-A1- 2004 265 238
- US-A1- 2005 165 340
- US-A1- 2005 271 596
- US-A1- 2006 110 413
- US-A1- 2006 275 229
- TIRAPELLI C R ET AL: "VITAMIN K ATTENUATES HYPOXIA-INDUCED RELAXATION OF RAT CAROTID ARTERY" PHARMACOLOGICAL RESEARCH, ACADEMIC PRESS, LONDON, GB LNKD- DOI:10.1016/S104366180200227X, vol. 46, no. 6, 1 December 2002 (2002-12-01), pages 483-490, XP001155846 ISSN: 1043-6618
- GOURGOU ET AL.: 'Lower Limb Venous Insufficiency and Tobacco Smoking: A Case-Control Study?' AMERICAN JOURNAL OF EPIDERMIOLOGY vol. 155, no. 11, 01 June 2002, pages 1007 - 1015, XP008130095
- Schurgers Leon: "MenaQ7 - Natural vitamin K2 - monograph", Oman Medical Journal, 4 June 2008 (2008-06-04), pages 172-177, XP055478792, Muscat - Oman DOI: 10.5001/omj.2014.44 Retrieved from the Internet: URL:https://pdfs.semanticscholar.org/f844/ 9e649856f09bd017d4b4432f4fb1a7e02404.pdf [retrieved on 2018-06-04]
- Leon J Schurgers ET AL: "Vitamin K-containing dietary supplements: comparison of synthetic vitamin K 1 and natto-derived menaquinone-7", , 7 December 2006 (2006-12-07), XP055480782, DOI: 10.1182/blood-2006-08- Retrieved from the Internet: URL:http://www.bloodjournal.org/content/bl oodjournal/109/8/3279.full.pdf?sso-checked =true [retrieved on 2018-06-04]
- CARIO-TOUMANIANTZ CHRYSTELLE ET AL: "Identification of differentially expressed genes in human varicose veins: Involvement of matrix GLA protein in extracellular matrix remodeling", JOURNAL OF VASCULAR RESEARCH, KARGER, BASEL, CH, vol. 44, no. 6, 1 January 2007 (2007-01-01), pages 444-459, XP008184167, ISSN: 1018-1172, DOI: 10.1159/000106189 [retrieved on 2007-07-20]

## Description

### FIELD OF THE INVENTION

The present invention is related to compositions for oral administration comprising a therapeutically effective amount of vitamin K2 either alone or in combination with one or more therapeutically effective active agents for use in the improvement of blood perfusion and the amelioration of hypoxia in specified chronic venous insufficiency and post thrombotic syndrome complications in human subjects, for use in the relief from the signs and symptoms of certain chronic venous insufficiencies and for use in the treatment of post thrombotic syndrome complications.

There is also disclosed the therapeutic uses of vitamin K, its analogues and derivatives and vitamin K activity synthesized molecules to improve blood perfusion and ameliorate hypoxia for the prevention and/or treatment of chronic venous insufficiencies and consequent diverse complications such as venous and lymphatic oedema, melanization, paper money skin, desquamation, restless leg syndrome, muscle cramps, purpuric petechiae, spider - web phlebomegaly, heaviness of legs, paresthesiae and lower limb nerve pain, pooling of blood in inferior limbs and reduced venous return, venous stasis, orthostatic intolerance, thrombophlebitis and thrombus formation in veins and embolism thereof resulting in cardio pulmonary vascular events. The principle extends similarly through microcirculatory dysfunction in other organs also such as lung, heart, liver, brain, kidney retina, pancreas, testis, rectum, spinal cord, muscles, sympathetic and parasympathetic ganglia, and nerves. Also disclosed is
administering vitamin K analogues for-improving skin, hair and nail health in subjects in need thereof. The invention further extends to pharmaceutical compositions of vitamin K, its analogues and derivatives either alone or in combination with active agents for use in the disease or conditions encompassed by the invention.

### BACKGROUND

In 1929, the Danish Nutritional scientist Dr. Henrik Dam discovered that feeding chicks a totally fat-free diet caused uncontrolled bleeding under their skin [1], Dr. Dam quickly discovered the reason for this disturbing effect: the diet was missing a previously unknown fat-soluble nutrient, which he appropriately named "koagulationsvitamin" - literally, the "clotting vitamin". The English name for the new vitamin was taken from Dr. Dam's Danish: "K", for "Koagulation".

Science was established that the gamma-glutamyl carboxylase is an integral cellular glycoprotein that uses vitamin K, a cofactor, to modify clusters of glutamyl residues (glu's) to γ-carboxylated glutamyl residues (gla's) post-translationally in vitamin K-dependent (VKD) proteins as they pass through the endoplasmic reticulum. Carboxylation is required for VKD protein functions in hemostasis. The VKD proteins, in the coagulation cascade, needing vitamin K to transact from the Glu status to Gla, to be biologically active, were proteins II, VII, IX, X, C, S and Z. It is interesting to note the surprising fact that the carboxylase itself has been shown to be a VKD protein [2].

For over fifty years, nearly everyone thought that the vitamin K story began and ended with blood clotting. If doctors paid attention to it at all, it was only to make sure that it didn't interfere with the blood-thinning, anticoagulant drug warfarin (Coumadin®). But even as nutrition textbooks and mainstream medicine continued to think of vitamin K as a one-act show, a paradigm shift had been forced onto researchers in the late 1970's when new vitamin K-dependent protein associated with bone - building osteoblast cells was discovered [3, 4]. Lian et al [4] named it osteocalcin.

**Vitamin K Analogues** - Vitamin K is, in fact, a family of structurally similar, fat-soluble, 2-methyl-1,4-naphthoquinones, including phylloquinone (K1), menaquinones (K2), and menadione (K3). The structural difference is in the substituent side chain at the gamma position (Schematic A).

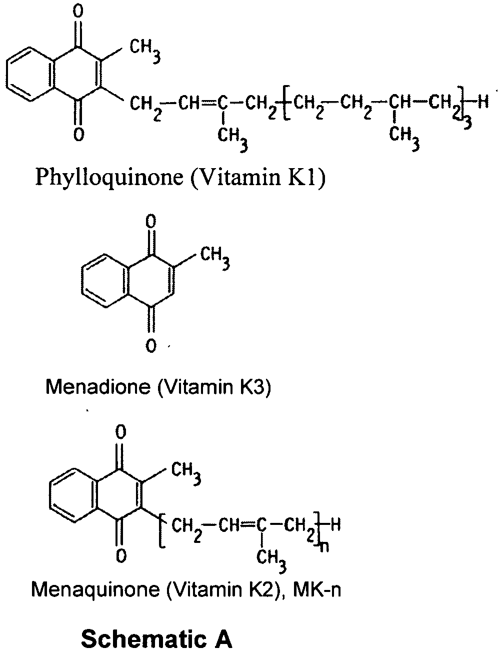

The best-known member of the vitamin K family is phylloquinone (K1), also known as phytonadione because of its relationship with photosynthesis. Phylloquinone is found in higher plants and algae, with the highest concentrations found in green leafy vegetables [5]. Vitamin K1.derived from the food intake is selectively distributed in hepatic and non-hepatic tissues.

Menaquinones (K2) also occur naturally, but are produced by an array of bacteria, generally not by higher plants, except for K2-4. Recent studies have determined menaquinones are produced in limited quantities by animals, and by humans, from the conversion of other forms of vitamin K [6, 7] The most common form of vitamin K2 in animals is menaquinone 4 (menatetrenone; MK-4), produced by the processing of exogenous and bacterial naphthoquinones [8]. Vitamins K1 and K2 differ only in the side chain in gamma position. Vitamin K1 possesses a phytyl group (partially saturated polyisoprenoid group) at position 3, while vitamin K2 possesses a repeating, unsaturated trans-poly-isoprenyl group. The IUPAC-IUB Commission on Biochemical Nomenclature abbreviates phylloquinone (K1) as "K" while menaquinone (K2) is abbreviated as "MK-n." The "n" signifies the number of unsaturated isoprene units that compose the side chain at the 3-position which may vary between 1 and 14. Here on we shall denote phylloquinone as PK and menaquinones as MK-n.

Menadione (K3) is not considered a natural vitamin K, but a synthetic analogue that acts as a provitamin. It possesses a much simpler structure, with no aliphatic side chain at position 3. Billeter et al [9] reported that phylloquinone can be cleaved to form menadione by bacteria in the intestine. Here on we shall denote menadione as K3.

**Biochemistry** - Only known function of vitamin K is that it serves as a cofactor for γ-glutamylcarboxylase, an endoplasmic enzyme involved in the posttranslational carboxylation of glutamate residues into γ-carboxyglutamate (Gla). Hence, the vitamin K-dependent step is a carboxylation reaction taking place during the later stages of protein biosynthesis. Vitamin K hydroquinone (KH₂) is the active coenzyme, the oxidation of which to vitamin K 2, 3 epoxide (KO) provides the energy to drive the carboxylation reaction. The resulting Gla-residues are found in a limited number of proteins, and in these proteins only at certain well-defined positions [10].Specific staining techniques or HPLC detection (after hydrolysis) identify the Gla-proteins as unique products of vitamin K action. In vitamin K-deficiency the carboxylation reaction cannot proceed, hence the Gla-proteins are released in an undercarboxylated form. Gla-residues form calcium-binding groups in proteins. So the main physico-chemical difference between normal and decarboxy proteins is their large difference in both binding of calcium from solution and the adsorption of these proteins to insoluble calcium salts.

**Bioavailibilty** - Booth et al [11] quotes Gijsbers et al [12] reported that the bioavailability (the area under an absorption curve) in human subjects of 1 mg phylloquinone in spinach was only 4% that of pure phylloquinone. Adding butter to the spinach increased this to 13%. Schurgers et al [13], through their experimental studies of PK absorption from spinach and MK-7 absorption from Natto, one week apart to exclude mutual interference of absorption, and also broccoli as source for PK and curd cheese and egg yolk as sources for higher menaquinones (MK-8 and MK-9) and MK-4 respectively, conclude that "*In all cases it was found that PK absorption from vegetables was very poor (5-10% without concomitant fat intake and 10-15% if taken together with 30 g fat), whereas menaquinone absorption from dairy produce and natto was much better, probably almost complete*." This fact makes a large section of the population susceptible to vitamin K deficiency, when these vitamins are lacking in the diet.

In absolute amounts PK forms well over 80% of the total amount of vitamin K in the human diet [13]. However, it must be noted that due to different bioavailability, MK's absolute absorption in the system will be higher. Considering 10% bioavailability for PK and 80% bioavailability for MK, ratio of PK/MK absorbed by the body is 8/16 i.e. 1 as to 2.

**Bioactivity** - Ushiroyama et al [14] state that compared to other vitamin K analogues, MK's have the most potent gamma-carboxylation activity.

**Pharmacokinetics** - Schurgers et al [13] studied pharmacokinetics of PK and MK vitamins. Their findings are that PK has an elimination half life of 1.5 hrs whereas the long chain menaquinones (but not MK-4) had more complex disappearance curves with a long half-life. MK-7 remains detectable even at 72 hrs and the concentration at that time is greater than the 50% of the peak value concentration, (Cmax), of that achieved by PK.

**Toxicity** - Even in high doses, the natural forms of vitamin K have not produced symptoms of toxicity. For this reason, the Institute of Medicine at the National Academy of Sciences chose not to set a Tolerable Upper Limit (UL) for vitamin K when it revised its public health recommendations for this vitamin in year 2000 AD. Consuming more than the body's needs for dietary vitamin K does not cause the blood to clot excessively in healthy people.

**Distribution and Metabolism** - PK is selectively distributed in hepatic and non-hepatic tissues; the heart contains as much as the liver but the brain has low concentrations in experimental rats. Thijssen et al [7], with a study in postmortem human tissues, have shown that in man there are tissue-specific, vitamin-K distribution patterns comparable to those in the rat. Ronden et al [15] comment that since the heart contains no gamma-glutamylcarboxylase, the function of vitamin K in this tissue remains obscure and is worth exploring. MK-4 is found in every tissue and is at a higher concentration than PK except in liver, where MK-4 has relatively low levels. MK-4 is nevertheless in large amounts in the exocrine organs such as the pancreas and the salivary gland. The brain also contains high concentrations of MK-4. Similar patterns of the tissue-specific distribution of members of the vitamin K group are observed in animals and humans [7, 16] The livers of chicks , irrespective of the fact that the sole source of vitamin K was PK, contained as much MK-4 as PK [17].

Gut flora produces higher amounts of MK-6 to MK-9 and very little MK-4. Therefore, MK-4 concentrations are not significantly different between normal rats and germ free rats. Further, MK-4 is hardly present in common food products. Thijssen et al.'s [18] observations that extra hepatic tissues contained more MK-4 in rats fed a PK-rich diet than in rats fed an MK-4-enriched diet; are consistent with the earlier results of germ free rats. Hence, it would appear that MK-4 may originate from the conversion of PK in the body [6, 19].

The speculation as to the variations has been put to rest by Okano et al [20] who, in an excellent study in mice with labeled and multi-route administration of menadione (K3) and PK, have shown that menadione is consistently converted to MK-4 irrespective of the route of administration..However, PK is converted to K3 following an oral or enteral administration, but not by parenteral or intracerebroventricular administration. This implies that PK is converted to MK-4 via integral side-chain removal in the intestine. In summary, their study "shows for the first time that MK-4 existing in cerebra of mice originates from PK and/or menadione intake, and suggest that there are two routes of cerebral MK-4 accumulation, one is the release of menadione from PK in the intestine, followed by prenylation of menadione into MK-4 in the tissue, and another is the release and prenylation of menadione both within the cerebrum."[20].

**Glu Proteins** - The discovery of osteocalcin brought forth the importance of carboxylation of Glu-osteocalcin in the body and the role of vitamin K. Osteocalcin is involved in calcium uptake and bone mineralization. Around the same time, it was also recognized that vitamin PK is more specific to carboxylation in the liver whereas vitamin MK is spread out throughout the body. Thus, vitamin MK has a greater importance as a cofactor in the carboxylation of osteocalcin. A number of clinical trials have proven the importance of vitamin MK supplementation for the bone health. Cockayne et al [21] have reviewed these trials in a meta-analysis and concluded that the supplementation with vitamin K reduces bone loss. Similarly, MGP (Matrix Gla Protein) has protective significance in the vascular calcification and resultant cardio vascular events.

**Epidemiology-** Epidemiological population-based Rotterdam study [22] examined the correlation between dietary intakes of vitamin K and aortic calcification and coronary heart disease (CHD). Their follow-up of 4807 subjects, with no history of myocardial infarction, over a period of 10 years showed a significant 50% reduction in CHD mortality and aortic calcification and 25% reduction in all cause mortality for those who were in the 45 µg per day, of dietary intake of vitamin MK-7 in the upper tertile and 25% reduction in 24 µg per day mid tertile compared to the 12 µg per day lower tertile. Such a population dose-response relationship was impressive for the new role of vitamin K.

The Japanese natto(soy) fermented food contains approximately 998 µg of vitamin K2-7 per 100 gms (882 to 1034 µg per 100 gms) [13]. Kamao et al [23] report vitamin MK-7 content of various fermented natto's: Natto(fermented soybeans) 939±753(µg/100g), Hikiwari natto (chopped natto) 827±194 (µg/100g), Black Bean Natto 796±93(µg/100g). He also reports that the typical intake of fermented natto in Japan is approximately 80 gms per day. Kaneki et al [24] report that serum MK-7 concentrations were 5.26 ±6.13 ng/mL (mean ± SD) in the Japanese women in Tokyo, 1.22 ± 1.85 ng/mL in the Japanese women in Hiroshima and 0.37 ± 0.20 ng/mL in the British women. Natto is eaten more frequently in Eastern Japan, Tokyo, but less frequently in Hiroshima and is not at all a food item in Britain. Kaneki et al [24] discovered a statistically significant inverse correlation between incidence of hip fracture, natto consumption and serum MK-7 levels between the three groups. Tskamoto et al [25], in a prospective study, administered 50 gms of natto per day to three groups. The concentration of MK-7 was either 865, 1295 or 1730 µg of MK-7 per 100 g. General population in Eastern Japan has 100 gms of natto for breakfast at a time. Tsakamoto [25] found that the serum MK-7 concentration and γ-carboxylated osteocalcin concentration were both elevated parallel to the administered level of MK-7.

**Other Uses** - Recent advances in the field of vitamin K have resulted in attributing novel functions for the molecule that are relatively less known. Traditionally, vitamin K has been associated with blood coagulation where it serves as a cofactor for the carboxylation of vitamin K-dependent proteins of coagulation cascade to render them active. It is now known that vitamin K is present in every tissue and by the virtue of its ubiquitous nature, the molecule plays an important role in bone mineralization, arterial calcification, apoptosis, phagocytosis, growth control, chemotaxis and signal transduction [26]. There is abundant literature and supportive data , highlighting the role of vitamin K in bone mineralization as in osteoporosis [21]. Recent studies have provided support on the role of vitamin K nutriture on atherosclerosis. However, for several tissues the role of vitamin K is yet to be discovered.

US20020015762 provides teachings of menaquinone addition to food products to promote human bone health and cardiovascular health. US20050123603 teaches pharmaceutical compositions and nutritional supplements with MK-7 for human health. Vermeer (US 20060166948) discloses arterial effects of vitamin K giving relief to age-related stiffening of the arteries. Consequently, the application claims, vitamin K for use in combating various cardiovascular events including hypertension, congestive heart failure and stroke. US20050176778 claims uses for osteoporosis and arterial calcification, with no coverage of chronic venous insufficiency.

US20050107472 teaches that naphthoquinone-type compounds including vitamin K can modulate aggregation of protein associated with neurodegenerative disease such as Alzheimer's disease. Furthermore, US20060058398 claims vitamin K to have an effect for the nervous system and an effect of potentiating nerve growth factor activity and treatment of diseases associated with dementia. However, there are no claims on the venous tonal health and perfusion effect of vitamin K on brain, spinal cord, sympathetic and parasympathetic ganglia and nerves.

Horrobin, in the US20020025983, claims that mixture of Essential Fatty Acid (EFA) and artificially elevated vitamin K have synergistic effect in treating and preventing a variety of diseases including menstrual disorders, osteoporosis , arthritis and mental disorders. But there are no claims on chronic venous insufficiency related problem.

### SUMMARY

According to a first aspect of the present invention there is provided a composition for oral administration comprising a therapeutically effective amount of vitamin K2 either alone or in combination with one or more therapeutically effective active agents for use in the improvement of blood perfusion and the amelioration of hypoxia in venous insufficiency and its manifestations in human subjects wherein the venous insufficiency is not varicose veins, for use in the relief from the signs and symptoms of chronic venous insufficiencies in human subjects, as a phlebodynamic agent; wherein the venous insufficiency manifestations are selected from the group consisting of paresthesiae, itching, cramps and heaviness of limbs; or for use in the treatment of post thrombotic syndrome (PTS) complications in human subjects, wherein the PTS complications are selected from the group consisting of heaviness, cramps, itching and tingling in limbs.

Also disclosed is a therapeutic method of use of vitamin K, especially PK and MK, their analogues and derivatives as well as novel vitamin K-like molecules that exhibit vitamin K activity, to improve blood perfusion and ameliorate hypoxia in prevention and treatment of certain disease conditions and abnormalities observed in subjects (humans) including chronic venous insufficiency with consequent diverse complications of venous, lymph and nerve abnormalities.

Also disclosed is the use of vitamin K and its analogues in improving blood perfusion and ameliorating hypoxia in venous insufficiency and its manifestations. Venous insufficiency manifestations are selected from the group consisting of varicose veins, oedema telangiectasiae, venous ectasia, hyperpigmentation, redness, paresthesiae, swelling, itching, cramps, lipodermatosclerosis, ulcers, Venous Thromboembolism (VTE), heaviness of leg and pain. Features of post thrombotic syndrome (PTS) are common to the symptoms which response to vitamin K, preferably MK-7.

Also disclosed is administering vitamin K analogues for the reduction in increased blackness of skin due to melanization. The conditions that exhibit such a reduction in melanin include but are not limited to freckles, age spots, melasama and Melanesia, and also paper - money skin, desquamation related roughness of the skin, puffiness due to oedema and dark circles around the eyes. Vitamin K and its analogues also provide relief in actinic and iatrogenic purpura, lentigines, spider angiomas, spider veins of face and legs and other vascular problems of skin and subcutaneous tissues when administered orally.

Also disclosed is the treatment of hyperpigmentation that arises as an associated symptom in diseases or conditions such as chronic venous insufficiency, diabetes (acanthosis nigra), scleroderma - and local melanization due to sunlight, UV exposure or mechanical irritation by administering vitamin K, its analogues, derivatives or vitamin K-like compound either alone or combination with one or more therapeutically effective active agents to enhance or facilitate their action..

Vitamin K analogues when administered as phlebodynamic agents may provide relief from the signs and symptoms of chronic venous insufficiencies. For example vitamin K treatment may be beneficial in relieving heaviness of legs, parathesiae (tingling and numbness sensation), cramps and stasis in varicose veins. Optionally vitamin K treatment can be extended to venous and lymphatic oedema, melanization, paper - money skin, desquamation, restless leg syndrome, purpuric petechiae, spider - web phelebomegaly, lower limb nerve pain, pooling of blood in the venous system and reduced venous return to the heart, venous stasis, orthostatic intolerance, venous thrombophlebitis, and thrombus formation in veins

Also disclosed is the prevention of muscle cramps in subjects by administering vitamin K analogues.

Therapeutic use of vitamin K analogues to increase the overall energy level, or in other words to reduce chronic fatigue is also disclosed.

The invention provides pharmaceutically effective doses of oral vitamin K analogues for prevention and/or treatment of disease conditions. Oral doses of vitamin K formulations can be effectively combined with topical application of vitamin K, for the skin-related uses, to achieve the desired effect. In certain instances, a cumulative effect is observed in the combination treatment regimen.

The invention further provides pharmaceutical compositions comprising vitamin K, its analogues, derivatives or vitamin K-like compounds either alone or in combination with one or more therapeutically effective active agents (as provided in Table 1) for the diseases/conditions that are encompassed by this invention.

### BRIEF DESCRIPTION OF DRAWINGS:

Figure 1: Effect of test samples PK-400µg/ml and MK4-400µg/ml on guinea pig ileum induced by 50 µg/ml of acetylcholine.
Figure 2: Effect of test samples PK-400µg/ml and MK4-400µg/ml on guinea pig ileum induced by 1% Barium Chloride.
Figure 3: Effect of test samples PK-400µg/ml and MK4-400uµg/ml on guinea pig ileum induced by 25µg/ml of histamine.
Figure 4: Effect of test samples PK-400µg/ml and MK4-400µg/ml on Frog rectus abdominus muscle contractions induced by 100µg/ml of acetylcholine
Figure 5: Effect of test samples PK-400µg/ml and MK4-400µg/ml on frog heart induced by 40µg/ml of adrenaline.
Figure 6: Effect of propanolol on adrenaline inhibition.

### DESCRIPTION OF THE INVENTION

various conditions for which therapeutic use of vitamin K analogues are encompassed by the present invention are disclosed in the foregoing discussion.

Any disclosures relating to therapeutic uses which fall outside of the scope of the appended claims are provided for reference only.

### Chronic Venous Insufficiency

The heart pumps blood through the arteries, arterioles, through a network of capillaries, than venules and veins. The capillaries have very thin walls that allow nutrients to pass through into the tissues and waste products to filter back into the capillaries. The venous tone and flow are important for return of blood back to the right heart, lungs and then to the left atrium and left ventricle.

Commonly the diseases affecting arteries and veins are often different. 'Atherosclerosis', plaques in the inner layer of aorta and arteries, narrows and blocks arteries with thrombosis, causing heart attacks, strokes and gangrene, but it does not cause problems in the veins. However, 'Thrombosis' means clotting of blood in a blood vessel and can occur in either veins or arteries, but the causes and consequences are different. Thrombosis in the deep veins of the legs can be quite dangerous (Thrombosis, phlebitis and embolism).

Chronic venous insufficiency is a syndromic term covering a wide variety of symptoms which may severely impair the patient's physical well-being or even lead to partial or complete invalidism persisting for years. Also involved on a large scale in these disturbances is the microcirculation. "Histangic"factor (interface between vessels and tissues) plays a basic role in the venous insufficiency and more in all venous disorders.

Evans et al in the Edinburg Vein Study [27, 28] have noted that telangiectasis and reticular veins were each present in approximately 80% of men and 85% of women. Varicose veins were present in 40% of men and 16% of women, whereas ankle edema was present in 7% of men and 16% of women. Active or healed venous ulcers occur in approximately 1% of the general population. These are often recalcitrant to treat. In the Framingham Study [29], the annual incidence of varicose veins was 2.6% among women and 1.9% among men. There is also a significant impact on the quality of life by the severity of venous disease. With age, the venous insufficiency gets aggravated.

It was serendipitously discovered that when vitamin K is administered, preferably vitamin MK-7, the features of the syndrome due to varicose veins diminishes. We also disclose vitamin K's therapeutic use in diseases involving the venous system of the lower extremities, testies (varicocele), rectum (haemorrhoids) and retina (venous tortuosity) and elephantiasis.

It is not obvious that it's the vasa vasora, vasa nervosa and vasa cutis, where these processes are the most important, result in restricting blood supplies temporarily and leading to plethora of symptoms that is observed and surprisingly resolve the symptoms to a great extent through administration of vitamin K. Vitamin K improves blood supply by improving blood perfusion and brings about homeostasis. Yet another improvement is muscle tone.

The current invention indicates the central role played by vitamin K in improving blood perfusion and ameliorating hypoxia in the venous insufficiency and its manifestations. Owing to microcirculatory disturbances, the damaged vessels produce a detrimental effect on the surrounding tissues. The therapeutically evidence fully supports the mechanism that clinical resolution is dependent upon vasculo-tissular (histangic) compensation by an improvement in venous tone and flow. The applicants claim histangic compensation of venous insufficiency which per se can lead to reverse cardiovascular and pulmonary events, due to reasons other than the earlier reported claims of calcification in the patent literature. Vitamin K can enhance tissue perfusion, microcirculation and relieves hypoxia by action on the venous system. Manifestations of venous insufficiency as meant here includes but not limited to oedema, telangiectasis, hyperpigmentation, redness, paresthesiae, swelling, itching, cramps, lipodermatosclerosis, ulcers, heaviness of limb, unsteadiness due to venous pooling and pain.

It should also be noted that Chronic venous insufficiency encompasses wide range of diverse symptoms. There are many diseases such as Diabetes Mellitus, Post thrombotic syndrome, Hypothyroidism, Scleroderma, Chronic renal failure, Hyperparathyroidism where similar symptoms can be noticed. Vitamin K is effective in ameliorating the symptoms through its ability to improve venous tone and flow. It is also the observed by the applicants of the present invention that vitamin K's effect can be further enhanced by several agents across various, disease conditions with similar symptoms. These active agents include but are not limited to the list provided in Table 1. Some of these agents are already used as conventional therapeutic for some of the conditions, e.g. vitamin B12, and its derivatives in paresthesiae. Typically, as per the; current invention, one or more of these agents are administered in combination with vitamin K for the disease/conditions encompassed by this invention.

**Table 1: Therapeutically effective Active Agents**

| Name of the Agent |
|---|
| Vitamin K |
| Glucofuranoside (Glyvenol) |
| Vitamin D2 and D3 |
| Vitamin B12 and its salts. (hydroxo, methyl and adenosil) |
| Folic Acid |
| Vitamin C |
| Vitamin E |
| MSM |
| Betaine |
| Warfarin, |
| L- Dopa Formulations |
| Ropinirole Hydrochloride |
| Horse ChestNut |
| Nattokinase |
| Trigonella foenum graecum Linn. (Fenugreek) |
| Bioflavonoids |
| Electrolytes |
| Quinine |
| Magnesium Citrate |
| Choline |
| Selenium |
| Baclofen |
| Coenzyme Q10(CoQ10) |
| Acetyl-L-Carnitine |
| Shilajit |
| Dehydroepiandrosterone (DHEA) |
| Phosphatidylserine (PS) |
| Melatonin |
| Omega-3,6 and 9 Polyunsaturated Fatty Acids, |
| Niacin |
| Inositol hexaniacinate (IHN) |
| Centella Asiatica, (Gotu Kola) |
| Hamamelis Virginiana (Witch Hazel) |
| Lipoic Acid |
| Linolenic Acid and α Linolenic Acid |
| Diosmin |
| Hesperidins |
| Calcium and it salts |
| Calcium Fructoborate |
| Idebenone |
| Riboflavin |
| Kinetin |
| Proanthocyanidins |
| Vitamin A |
| Lactobacillus GG (Probiotics) |
| Bacillus Subtilis |
| Bacillus Licheniformis |
| Yeast sacc. Cerevisiae |
| Yeast sacc. Boulardii |
| Celery and its derivatives |

### Cramps

Chronic, persistent leg cramps are a common and distressing problem. Christine Roffe et al [30] quotes that the prevalence of leg cramps increases with age, affecting 30% of >60 year olds and 50% over the age of 80 [31, 32]. Cramps may be precipitated by changes in water and electrolyte homoeostasis or by drugs such as diuretics, laxatives, beta2-agonists, cimetidine, and phenothiazines. But the fundamental mechanism of cramp is not well understood. That is why the adjective "Idiopathic" is used.

Idiopathic nocturnal cramps are painful involuntary muscle spasms that commonly disrupt sleep. More than a third of people aged over 60 years experience them, their prevalence increases with age and they occur most commonly in the leg [31, 32]. Such a wide spread distress has no adequate therapeutic intervention. There are six basic causes of cramping: hyperflexion; inadequate oxygenation; exposure to large changes in temperature; dehydration; low blood supply; or low blood calcium. Most of muscle cramps are due to hypoxia and inadequate perfusion.

Current modalities commonly used in the treatment of muscle cramps are electrolyte supplementation and muscle relaxants. These treatments frequently fail to produce desirable and lasting effects. We have discovered that vitamin K daily administration not only relieves but also prevents recurrence of cramps. Daily dose of vitamin K needs to be titrated as per the severity and frequency of cramps.

Half life of vitamin MK-7 is high (MK-7: 50 hrs) [13]. So MK-7 can be gainfully used due to its presence in the body for a longer duration. Thus the invention also provides the use of vitamin K as a safe prophylactic for muscle cramps. Vitamin K also improves the muscle strength as evident by relief of fatigue.

The inventors have discovered relief from cramps when a sufficient dose of vitamin K is administered systematically daily once or more. Dose will depend on the vitamin K analogue and the pharmacokinetic profile of the particular analogue used for the treatment. The preferred range is 10 µg to 1000 µg per day, and the preferred vitamin K is vitamin MK-7. We have found that vitamin K reduces and even eliminates condition of cramps.

### Hyperpigmentation

The formation of melanin or the process of melanogenesis is based on the enzymatic conversion of the amino acid tyrosine, through a series of intermediates, to melanin pigments - the reddish brown pheomelanins or brown black eumelanins.

The tyrosinase enzyme is regarded as the key rate limiting enzyme involved in melanin synthesis Tyrosinase enzyme is a bifunctional enzyme in that it catalyzes the oxidation of tyrosine into L-DOPA (3,4-dihydroxyphenylalanine)̵and further catalyzes L-DOPA into DOPA-quinone. DOPA-quinones are transformed, through a series of intermediates, to reddish or brown-black polymeric melanin components that are responsible for skin pigmentation. Copper, in the bivalent Cu⁺⁺ state, is part of metalloenzyme - tyrosinase.

Tyrosinase is widely distributed in microorganisms, animals and plants and is a key enzyme in melanin biosynthesis, involved in determining the color of mammalian skin and hair. The sequence of melanin formation from tyrosine by the action of tyrosinase is depicted by Raper. [33] and Mason [34]. The control, modulation and regulation of the tyrosinase enzyme thus play a key role in melanin synthesis and skin pigmentation. Tyrosinase inhibitors are widely used in cosmetic, cosmeceutical and pharmaceutical products for their skin-whitening effects.

Skin-whitening agents are widely used to even out skin dark tone, and to depigment hyperpigmented skin, such as melasma, freckles, or age spots. Ultraviolet light stimulates melanocytes, producing greater quantities of melanin contributing to hyperpigmentation.

Common skin lightening/whitening agents used include hydroquinone, kojic acid, vitamin C and its derivatives, turmeric extract, and extracts of natural plants such as rumex and licorice. Natural compounds and vitamins are considered a good choice as pharmacological ingredients due to their relative freedom from side effects. Although the efficacy of some of these products has been demonstrated, in several cases mechanism of action is unknown or unconfirmed. Most skin whitening agents work by inhibition of tyrosinase enzyme but other mechanisms such as reduction of melanoblast or destruction of melanocytes is also possible. Inhibition of tyrosinase enzyme may vary among skin whitening agents. The said compound may either interact directly with the tyrosinase or control its activity indirectly by complexing with copper ions or bring about the said lightening effect by regulating calcium ions. [35, 36]

US5510391 provides the use of vitamin K mixture as a topical application for the treatment of blood vessel disorders of the skin including actinic and iatrogenic purpura, lentigines and other vascular problems of the skin and subcutaneous tissue. Furthermore, Shah et al [37] have described the effect of topical vitamin K on bruising after laser treatment.. This study particularly elaborates the beneficial effects of topical application of vitamin K after the laser therapy. Shah et al's study has been challenged by Kovacs et al [38] who have indicated a lack of effect of topical vitamin K on bruising after mechanical injury . They differ from Shah et al in their finding and conclude that vitamin K cream application in post laser bruising did not help in the cleaning of petechiae. In all of the above studies, vitamin K has been administered topically, and not systemically.

It is the objective of the skin lightening/whitening agent to interfere with the melanin synthesis or enhances its catabolism. The mechanism of action underlying the present invention is that the molecules with vitamin K activity, including PK and MK, activate Glu proteins through carboxylation and conversion of Glu to Gla, in the vicinity of the melanocytes. These Gla proteins interfere with several bivalent metallic ions involved in the biopathway of the melanin formation.

De Boer-van den Berg et al [36] have reported carboxylase enzymes in skin of humans, rats rabbits, and mice. Carboxylase converts Glu proteins to Gla proteins and are known to interact with bivalent ions, particularly Ca⁺⁺ions. It is reported that the carboxylase enzymatic activity in skin is about 20% of that in liver. The enzymatic activity present in both the epidermal and dermal tissues, may be related to melanin metabolism..

The inventors of the present invention have observed lightening of the skin and even disappearance of hyperpigmentation when a sufficient dose of vitamin K is administered systematically daily once a day or preferably twice a day. Dose will depend on the vitamin K analogue and the pharmacokinetic profile of the particular analogue used for the treatment. The preferred range is 10 to 1000 µg per day, and preferred vitamin K is vitamin MK-7. Furthermore, the applicants observe that systemic vitamin K-provides relief of actinic and iatrogenic purpura, lentigines, spider angiomas, spider veins of face and legs and other vascular problems of the skin.

Hyperpigmentation is an associated symptom in many of the diseases like, chronic venous insufficiency, Diabetes, Scleroderma etc. The most common factor attributed to this condition is poor tissue perfusion. The applicants have found that vitamin K reduces and even eliminates condition of hyperpigmentation, which is generally due to poor perfusion.

### Varicose Veins - for reference only

The veins of legs affected are either the superficial veins or the deeper veins. The superficial veins arc the ones that can become varicose. The superficial veins and deep veins are linked in a number of places by perforating veins (or 'perforators') and they are equipped with valves which when function improperly, then blood is pushed out into the superficial veins when the muscles contract: this is one reason for high pressure in the superficial vein and can be a cause of varicose veins. The varicose veins are not just limited to legs but can also be found in testes, rectum and retina. Both the poor venous tone and impaired valves reduce the upward venous blood flow toward the heart.

It was serendipitously discovered that when vitamin K is administered, preferably vitamin MK-7, varicose veins and its pain diminishes. The applicants also claim vitamin K's therapeutic use in diseases involving the venous system of the lower extremities and varicose veins of the testes, rectum and retina

### Desquamation

The skin consists of two primary layers: the deeper layer called the dermis, composed largely of adipose and connective tissue, and the superficial layer called epidermis. The epidermis contains cells that determine skin color and the pigment that protects from damage. Epidermal keratinized cells are constantly being worn away and replaced by new epidermal cells. In normal circumstances, injury to this layer of skin rarely causes problems because it usually repairs itself so quickly. For a person with some organic diseases like, diabetes, however, this can be a problem because once the outer layer of skin is atrophic and if tampered with; it may not heal as quickly or normally as in healthy subjects.

The epidermis consists of five *strata*; from outer to inner they are: the stratum corneum, stratum lucidum, stratum granulosum, stratum spinosum, and stratum basale. The deepest layer of the epidermis, the stratum basale, is a single layer of cells resting on a basement membrane (layer between the dermis and epidermis). The stratum basale cells divide continuously. As new cells form, older ones are pushed towards the skin surface. Those cells die that are pushed away from this layer. When the cells reach the skin surface, they are sloughed off in a process called desquamation. The stratum granulosum, the thin middle layer, initiates keratinization (production of keratin). This process starts the death of epithelial cells.

During desquamation, keratinocytes are pushed towards the surface. When these cells reach the outer layer of epidermis, they are keratin-filled. Millions of these dead cells are worn off daily, creating a new epidermis every 35 to 45 days.

There are many modalities available for the treatment of this particular problem, but yet they fail to achieve the complete cure i.e. smooth skin of heel and are also reported to have side effects. Some of the non medicinal treatments for desquamation include application of a moisturizing cream, use of a pumice stone to reduce the thickness of the hard skin, avoiding wearing open backed shoes or thin soled shoes, wearing shoes with a good shock absorbing sole. Medications such as, retinoic acid (isotretinoin, tretinoin, acitretin, tazarotene, etc.), zinc pyrithionate and methyl athyl sulfate, sucralfate, for moist skin desquamation by radiation, carbohydrate derivative, β- hydroxy acids and salicylic acid, ceramides are also suggested to provide relief for desquamation.

As per the present invention, vitamin K and preferably MK-7 plays a major role in reducing desquamation. That is noted in several conditions and in aging. The applicants have found that vitamin MK -7 relieves and resolves desquamation conditions whether the patient is suffering from diabetes or other causes. Use of MK-7 on regular basis in sufficient quantity, leaves the skin smooth at the heel where there was earlier rough and exfoliating skin.

### Fatigue

In blood vessels, the function of Gla-proteins are associated with the local inhibition of thrombosis, inhibition of mineralization and stimulation of normal cell growth and prevention of apoptosis in growth arrested cells. Role of vitamin K as a therapeutic in cardiac conditions by improving microcirculation at the level of vasa vasora is also encompassed by the present invention.

Chronic fatigue is a common symptom of several chronic metabolic and neuromuscular disorders. The major determinants of fatigue are the perfusion - dependent reduced oxygen supply, inadequate supply of energy for actin - myosin interaction, from glycolysis, and higher lactate level in muscles.. According to the present invention, MK-7 relieves fatigue by improved perfusion and increased oxygenation and energy supply.

### Paresthesiae

Paresthesiae is common in many diseases including diabetic neuropathy. Tingling/numbness, burning feet, causalgia and proprioceptive imbalance are more commonly arises due to diabetes mellitus, aging and smoking/tobacco intake Common and often successful modality is administration of vitamin B12. Though vitamin B12 reduces the tingling and numbness in the extremities, there can be still residual numbness and tingling which can be quite irritating. There is no known remedy for this and it remains as a problem.

The applicants have found that MK-7 is very effective in overcoming the residual neuropathy in the extremities after administering cyanocobalamine and its derivatives. Furthermore as per the present invention, it is seen that in patients who are on vitamin B12 medication, administering vitamin MK-7 relieves numbness and tingling sensation more rapidly.

When vitamin B12 levels fall in the body, for example, due to Metformin in diabetics, there is appearance of numbness and tingling. At this point, if either dose of vitamin K or dose of vitamin B12 is increased then the symptoms of numbness and tingling disappear.

The applicants herein claim the use of vitamin K alone or with vitamin B12 for reducing and eliminating the condition of paresthesiae arising due to several causes, by an improvement in perfusion and stabilizing membrane potentials.

### Thrombophlebitis and Venous Thromboembolism (VTE), Venous stasis

Through the studies presented in the instant specification, the applicants indicate that as endothelial function deteriorates with aging and diabetes this phenomenon in turn changes the venous tone and reduces venous flow, leading to venous stasis. This is an antecedent of hypoxia and inflammation, thrombophlebitis may ensue with a chance of embolization which can be life threatening.

Current invention is an improvement by vitamin K in venous tone, resulting in improvement of venous perfusion and flow resulting in diminishing of stasis, and chance of inflammation and thrombosis. The hazards of venous thrombosis and embolism during air travel can be controlled by MK-7.

### Post Thrombotic Syndrome (PTS)

PTS is a corollary to Venous Thromboembolism (VTE). PTS is a chronic condition that develops in 20-50% of the patients after Deep vein thrombosis (DVT) [39-42]. Patients with PTS have persistent venous insufficiency and experience pain, heaviness, swelling, cramps, itching, or tingling in the affected limbs. These symptoms are further enhanced by standing or walking and relieved with rest, leg elevation and lying down. Kurz et al [43] mention that "*Signs that may be noted on physical examination of the limb include oedema, telangiectasias, hyperpigmentation, eczema, varicose collateral veins and, in severe cases, lipodermatosclerosis and ulceration"*

These features of PTS are common to the symptoms which respond to vitamin K, preferably MK-7. The beneficial effects are modulated by an improvement in the venous tone and flow due to better endothelial function.

### Oedema

The current invention indicates the central role played by vitamin K and its analogues in improving integrity of the capillary wall and tonicity of veins and lymphatic's. In one embodiment, the invention provides that vitamin K and its analogues especially vitamin MK-7 act on smooth muscles and help in reducing oedematus condition by improved fluid drainage by lymphatic and venous return.

Generation of interstitial fluid is dependent on the balance of osmotic pressure of the plasma and of hydrostatic (intravascular) pressure which act in opposite directions across the semipermeable capillary walls. Consequently, anything that increases oncotic pressure outside blood vessels or reduces oncotic pressure in the blood will cause oedema. Increased hydrostatic pressure inside the blood vessel will have the same effect. If the permeability of the capillary walls increases, more fluid will tend to escape out of the capillary, by venous and lymphatic stasis.

In another embodiment the invention provides evidence for inadequate perfusion that plays a key role in the mechanism of development of oedema which can be due to many causes. MK-7 helps in improving tissue perfusion and thus can reduce the oedematus condition. The current invention thus provides vitamin K as a therapeutic for lymphatic and venous oedema.

### DEFINITIONS

As used herein, the term "PK" refers to phylloquinone (K1) or vitamin K1, also known as phytonadione because of its relationship with photosynthesis.

As used herein, the term "MK-n" refers to Menaquinones (K2) or vitamin K2, abbreviated as "MK-n." The "n" signifies the number of unsaturated isoprene units that compose the side chain at the 3-position which may vary between 1 and 14.

As used herein, the term "K3" refers to menadione or vitamin K3.

As used herein, the term "Glu" refers to glutamyl residues in uncarboxylated VKD proteins.

As used herein, the term "Gla" refers to γ-carboxylated glutamyl residues (gla's) post-translationally in VKD proteins.

As used herein, the term "VKD" refers to Glu proteins that require carboxylase along with cofactor vitamin K to carboxylate to Gla protein.

As used herein, the term "vitamin K" refers to any molecule having vitamin K activity whether natural, e.g. PK, MK-n, or synthetically derived or analogues, derivatives or vitamin K-like compound.

As used herein, the term "histangic" refers to morpho-functional connections existing between vessels and tissues.

By 'combination' it is meant that vitamin K is administered with one or more of active agents that are conventionally used for a disease/condition encompassed by the current invention. Administration of the active agent(s) in combination with vitamin K may be simultaneous or sequential. Illustrative lists of active agents are provided in Table 1 of this specification.

Therapeutically effective active agents as used herein means, those agents that are therapeutically effective and conventionally or unconventionally used for diseases or conditions encompassed by the current invention.

The invention is more fully understood by reference to the following examples. These examples should not, however, be construed as limiting the scope of the invention. Rather, the scope of the invention is defined by the appended claims.

Examples 1-10 enumerate clinical case studies date and examples 11 and 12 provide *in vitro* experiments.

Any examples which fall outside of the scope of the appended claims are provided for reference only.

### CASE STUDIES

### Example 01

Female, age 64years, a known case of type 2 diabetes mellitus of 14 years duration complained about increasing darkening of the skin on the nape of the neck and also little below the neck region. The skin hyper pigmentation was noticed by her 3 years ago. She was disturbed about her cosmetic discomfort as she could not wear low neck tops. She has been a fairly we controlled diabetic taking Metformin 1000mgs twice daily, Dionil® twice daily, Aspirin 100mg per day and vitamin B-complex. She started taking MK-7 one and a half years ago. Gradually the hyper pigmentation started clearing and now it has almost subsided. In between in this year and a half either she had discontinued when she would not have received the vitamin almost for a month or more. During these periods she had noticed reappearance of the pigmentation in a milder form. Currently she has been on the MK-7 in a dose of 100 µg per day for now 2 months. However tingling and numbness had been her main complaints. During the periods when she is on MK-7 she gets a major relief from tingling and numbness. During periods of discontinuation of the vitamin tingling reappears within a couple of days. Not only these complaints are noticeable by her but they are also very disturbing as often there is a fair degree of burning of feet along with tingling and numbness. Currently she has complete relief from tingling, numbness and burning of feet.

### Example 02

Male, aged 70, a physician and a clinical pharmacologist by profession with a history of type 2 diabetes mellitus for four years and hypertension for fourteen years complained of the following (a) Tingling and numbness of both the feet, intermittently for one year,(b) muscle cramps, at night, waking him up from sleep, 2 to 3 times in a week and (c) thinning of the skin of the lower limbs , with easy traumatibility and paper - money skin changes in the area below the inner malleolus. He was on standard antihypertensive drug (Candesartan 8mg AM and 4mg PM), Metformin (500-250-250 mg) and Centrum® once a day. MK-7 was taken p.o. every day. The severity and frequency of muscle cramps steadily decreased over the next two weeks. The frequency of mild cramps dropped to one or two in a month over the next four months. The tingling and numbness reduced markedly over the period. The paper-money skin appearance was much reduced. Then MK-7 was discontinued for a month and gradually all the features described above returned with the same basal severity and frequency. MK-7 was restarted in the same dose schedule. Relief of the features was again experienced as before. The same dose was continued for a year. Meanwhile the subject was shifted to higher dose of Metformin (1000, 250, 250mg.) and developed vitamin B12 deficiency (MCV=105). Although tingling and numbness returned, muscle cramps stayed controlled along with paper-money skin. After injections of vitamin B12 (500 µg) over 2 weeks, there was a significant relief in tingling and numbness again. The subject also reported higher energy levels and less fatigue with added MK-7 to his schedule. There were no major changes in diet or exercise during the period.

### Example 03

Female, aged 59 yrs, with Height of 5'5" and weight of 60 kgs, on strict vegetarian diet. For many years of at least 30 years and above she had been experiencing severe cramps during the night. These cramps used to be experienced any time during the night which would make her get up from the sleep due to severity of pain. The conditions was such that normally she had to wake up her husband to massage the area where the cramps were experienced, and then after several minutes pain used to subside. She had to be extremely careful about her body movement during sleep especially stretching of legs. There were times when similar situation used to occur more than once during the night. Sometimes the pain used to subside by walking around the room and in the corridor. She was experiencing similar condition many times during the day also especially during long walks in shopping malls. The condition used to be more severe or aggravated if there was even slight over exertion like longer walk or standing for a long time. She started taking MK-7 100 µg one tablet per day about one year ago. After taking MK-7 within few days the incidence of cramps started to reduce. She has also experienced that whenever she had cramps, the duration and the severity of pain were reduced. Within 2 to 3 months it appeared that the cramps have already gone. After taking the tablet for six months the cramps had disappeared. However after that she stopped the tablet and within two weeks the cramps reappeared, but at this time the severity was relatively less. She again restarted taking doses regularly. Her cramps had disappeared again. Now she is on regular dosage of the tablet i.e. one every day and she experienced practically no cramps during the night or during the day. She concluded that regular usage of MK-7 tablets has definitely helped her during time of cramps.

### Example 04

Male, ages 68 years with hypercholesterolemia (Lipid Profile; 200 mg - Total cholesterol) for which he is taking Atorvastatin 5 mg one per day and 75 mg of Aspirin one per/day since last five years. At the age of 64 undergoes angioplasty. He is also having mild blood pressure. He complained of having irritating rough skin on both the heels. He started taking daily dose of vitamin MK-7 200 µg. Within 10-15 days he observed that, his skin had completely lost roughness and became smooth.

### Example 05

Female, aged 70, a gynecologist with a history of hypertension for nine years complained of paper-money skin and hyper pigmentation changes in the lower limbs. She has mild to moderate varicose veins. She has been on antihypertensive drug (Candesartan, 8mg PM), Atorvastatin 10mg and Aspirin-50mg once a day. She also took vitamin B-complex off and on. She was a known case of Primary Hypothyroidism and has been on Thyroid replacement therapy for more than 25 years. Vitamin MK-7 was taken p.o. every day in 100 µg per day dosage. Hyper pigmentation and the paper-money skin gradually reduced to a bare minimum. On discontinuation for a few months the skin changes reappeared. She went back to taking of MK-7. Within a few days time she started observing diminution of the pigmentation. She continues to follow life style where there is a daily walk of half an hour and avoidance of heavy fried or dense food. There were no major changes in diet or exercise during the period. Currently she continues to take the MK-7 in a daily dose of 100 µg per day. She had also experienced smoothness of skin of her feet, diminution of cracks in the feet and scalp hair-loss has stopped distinctly. These changes were observed independently and verbalized by her beautician also.

### Example 06

Mrs. S.S. aged 78 years had complaint of severe leg cramps for the past 10 to 12 years. She described that her pain in legs was excruciating that accompanied her leg cramps. Frequency of the cramps was 4 to 5 times in a day and 2 to 3 times at night. These cramps were of long duration, between 10 to 30 minutes, and time gap between recurrences was variable. These cramps woke her up from her sleep. Her daughter described this vividly. She said that her mother had to stretch her leg this way and that way but nothing relieved her cramps. She said usually a cramp gets terminated once you flex/extend the cramped part and change positions or cover with a blanket or so. But none such measure or pain killers or multivitamin or drinking electoral (hydrating fluids) relieved her severe cramps. On 12^{th} April 2007 she started taking MK-7 in a dose of 100 µg two times a day. Some positive effect was noticed in the severity of the cramps during the first nine days but cramps were present. After this for three days there were no cramps but very next day when there was physical strain (climbing of stairs, long time standing and had to walk) she relapsed into two days with severe cramps in both the legs for half an hour at a stretch. From here on cramps occurred off and on until 5th of May 2007. After the 5^{th} May there were no cramps except occasionally when there was severe physical strain due to climbing of the stairs or walking long distances or standing for a long time. As she continued taking MK-7 not only the frequency and duration as well as the severity of her cramps decreased but also triggering factors (long time standing, climbing stairs etc.) had less impact. She had taken the vitamin till August 2007. After stopping MK-7 there were no cramps for the next six days. After that the cramps restarted but with less severity, time duration, and there were no more than 4 times in 30 days. She was more than happy to have obtained MK-7 again and restart at the end of 30 days and continues with a dose of 100 µg two times a day and enjoys cramp free days and nights.

### Example 07

Sixty Four year old, female Homoeopathic Physician had complained of darkening of skin of her face and exposed parts of her arms and neck about 3years ago. This hyperpigmentation deepened over time and she noticed that burning and itching got worse as the pigmentation increased. From March 2007 she felt cosmetically so compromised as to lose confidence and felt embarrassed and socially defendant. She has not had any chronic disease like diabetes mellitus or hypertension. She has been having chronic insomnia for several years. There is no history of any medication other then occasional intake of homeopathy medicine for minor complaints of cold or cough. She took homeopathic medicine for 4months for her hyper pigmentation. She noticed that though burning and itching had decreased there was no change in her hyper pigmentation. At this stage she received MK-7 in a dose of 100 µg twice a day. Hyper pigmentation decreased after 2 to 3 months of intake of the vitamin. She continued the medication for another 3 months and noticed that there was considerable lightening of the skin. She stopped taking the vitamin after a total duration of intake of 6 months. Pigmentation darkened gradually once again on withdrawal of MK-7. Currently she is on the medication again for the past one month where she has found a noticeable change in her skin with lightening of hyper pigmented areas.

### Example 08

Female, age 32 years ethnic American, with no other known medical conditions except for cramps. She is an athlete and jogger and runs marathon. She would experience cramps few hours after the marathon. In March 2008, we put her on 200 µg per day of MK-7. After starting on treatment within 2 months, she observed that her severity of cramps was lowered. Serendipitously over a period of 2 months, she also observed that her nail grows faster and her trimming time reduced. Further she found similar effect in the hair growth.

### Example 09

Female aged 68 yrs, with a history of colitis at the age of 17 yrs and got cured at the age of 18 by nature-cure treatment. Had undergone 3 caesarian sections in the year 1970, 1971, and in 1972. In the year 1979, she was suffering from intestinal obstruction because of which she could not have even consumed 2 table spoon of water, and got operated. In the year 2000 again operated for gall Bladder. In the year 2006 February, detected to have Intestinal T.B. Detected as a diabetic in 1997 and then onward on the glycol red plus three times a day. She is also taking two Tab. Nurobian per day and one tab. of folic acid per day. She was experiencing the symptoms of residual neuropathy such as tingling, numbness, paresthesiae, burning sensation. MK-7, 100 µg per day: within 15 days reduces gradually and disappears completely. MK-7 was stopped after 2 months and was under observation then after. She observed that tingling and numbness returned within 1 week.

### Example 10

Male aged 64 complaining of angina problem controlled by Nattokinase and found that there was good control in Hypertension as well as in cardiac pain. He is taking Nattokinase form last three years two capsule per day 50 mg. He takes general vitamins from last 10 years suffering from restless legs syndrome every night and sometime during the day also. He used to get attack of restless legs syndrome at any time. He then started taking MK-7 200 µg per day from last 1 year. After starting with MK-7 within 2 weeks restless legs syndrome reduced considerably and now it is once or twice a month.

### Pharmacological Antagonism: in vitro studies in smooth and skeletal muscle

### Example 11

### Muscle Relaxant activity against known agents

Muscle relaxant activity was evaluated of pure test samples PK and MK-4 with Frog Rectus Abdominus Muscle and Guinea Pig ileum.

### Methodology

### A) Guinea Pig ileum

To evaluate the effect of test samples on smooth muscle contraction induced by (a) Acetylcholine (Neurotransmitter) which acts on nicotinic receptors, (b) Histamine that causes a transient rise of cytosolic calcium, which is mediated by HI receptor coupling that triggers release of internal calcium stores and (c) Barium that enters the cell through a voltage dependent calcium channel and, either directly or indirectly, interacts with calmodulin.

### B) Frog Rectus Abdominus Muscle Preparation

To evaluate the effect of test samples on skeletal muscle contraction induced by Acetylcholine (Neurotransmitter).

### A) Guinea Pig ileum

### Acetylcholine: 50 µg/ml

### Test Samples were PK - 400 µg/ml and MK-4 - 400 µg/ml

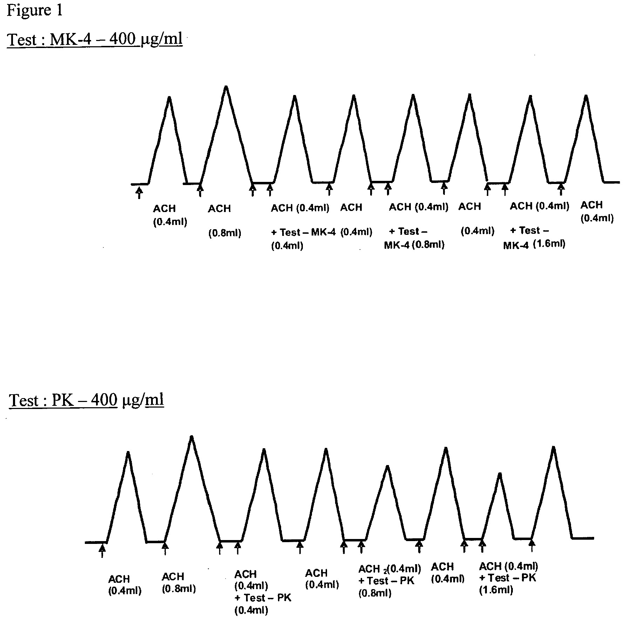

**Conclusion**: The compound MK-4 exhibited 21.62%, 40% and 81.81% inhibition of acetylcholine induced smooth muscle contraction at the dose of 80 µg (0.2 ml), 160 µg (0.4 ml) and 320 µg (0.8ml) respectively. The compound PK exhibited 38.46% and 46.15% inhibition of acetylcholine induced smooth muscle contraction at the dose of 320 µg (0.8ml) and 640 µg (1.6ml) respectively (Figure 1).

### Barium Chloride: 1 %

### Test Samples were PK - 400 µg/ml and MK-4 - 400 µg/ml

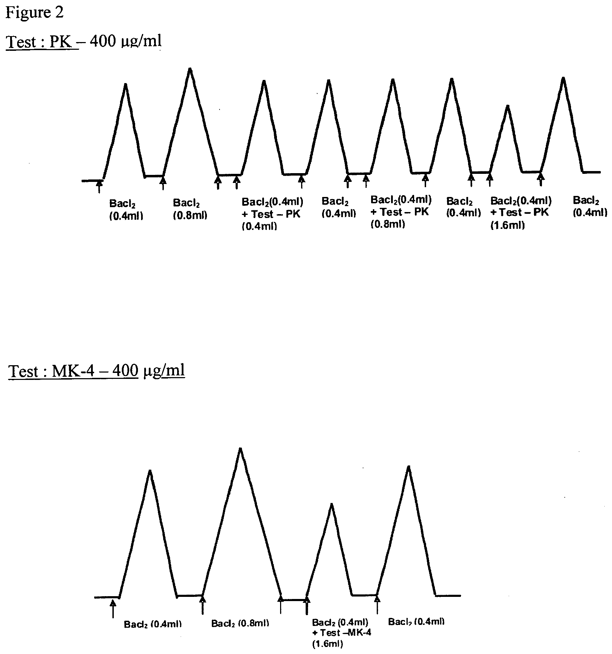

**Conclusion:** Test sample PK and MK-4 exhibited 31% and 29% inhibition of barium chloride induces smooth muscle contraction respectively at the dose of 640 µg (1.6ml) (Figure 2).

### Histamine : 25 µg/ml

### Test Samples were PK - 400 µg/ml and MK-4 - 400 µg/ml

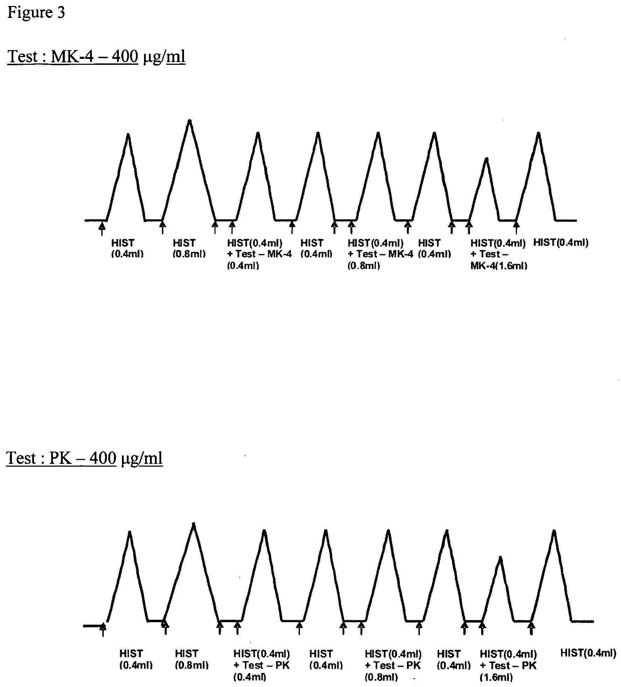

**Conclusion:** The test compounds MK-4 and PK exhibited 29.4% and 37.16% inhibition of histamine induced smooth muscle contraction respectively at the dose of 640 µg (1.6ml) (Figure 3).

### B) Frog Rectus Abdominus Muscle Preparation

### Acetylcholine: 100 mcg/ml

### Test Samples were PK - 400 µg/ml and MK-4 - 400 µg/ml

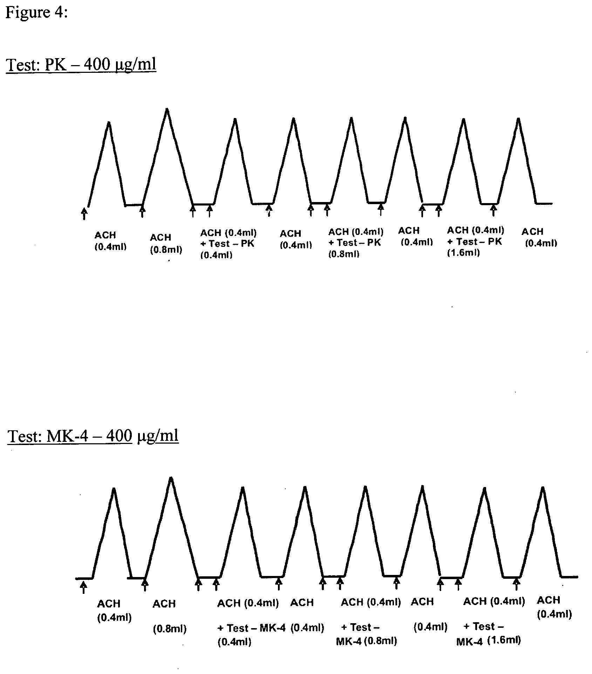

**Conclusion:** Contraction induced by acetylcholine (40 µg) was not antagonized by PK and MK-4 at the dose of 400 µg. As 80 µg of Acetylcholine could only marginally increase the amplitude of contraction as compared to 40 µg of Acetylcholine. This *in vitro* study may not be extrapolated to *in vivo* situation, as vitamin K being a fat soluble substance. Refer to Figure 4.

### Example 12

### Frog Heart

### Adrenaline: 40µg/ml

### Conclusion:

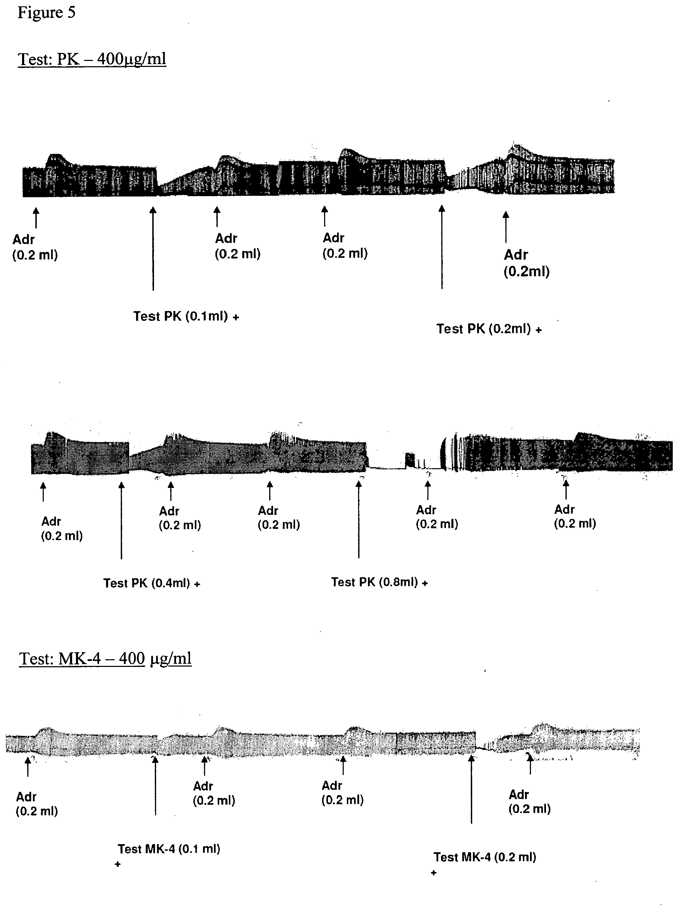

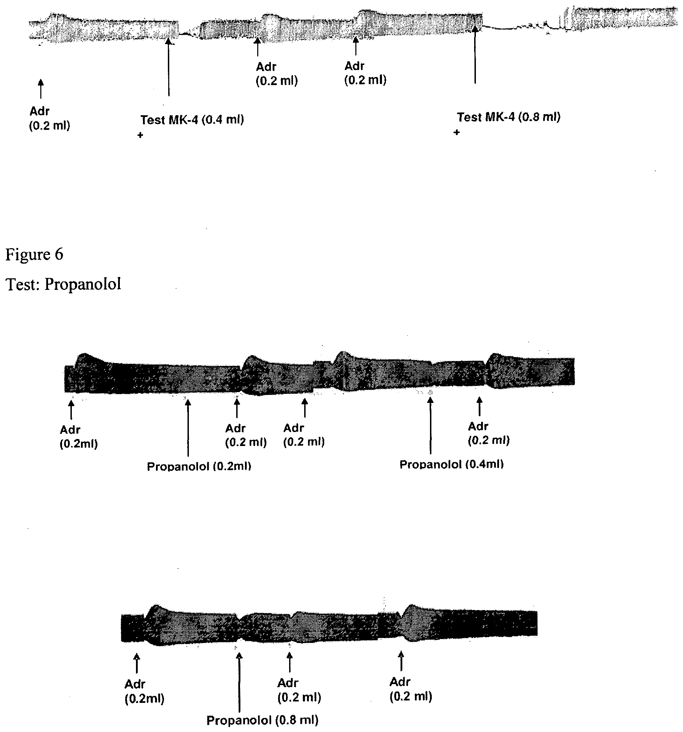

The test sample PK at 0.1ml (40 µg), 0.2ml (80 µg) and 0.4ml (160 µg) failed to inhibit adrenaline-induced contractions (Figure 5). However Propanolol a competitive inhibitor of adrenaline was found to inhibit adrenaline response (Figure 6).

The test sample MK-4 at 0.1ml (40 µg), 0.2ml (80 µg) and 0.4ml (160 µg) failed to inhibit adrenaline induced contractions (Figure 5). However propanolol a competitive inhibitor of adrenaline was found to inhibit adrenaline response (Figure 6).

### Inference:

• The data accumulated reveal that the test compounds PK and MK-4 demonstrate a smooth muscle relaxant activity.
• However, both the compounds failed to exhibit activity on skeletal muscle contraction.
• The test samples did not show inhibition of the adrenalic (beta receptor agonist) response, which indicated that both PK and MK-4 do not exhibit beta blocker activity. Thus suggesting that the peripheral perfusion effect is not due to a reduction in the arteriolar tone viz. decreasing peripheral resistance.
• Mechanism of action studies revealed that both PK and MK-4 could be demonstrating muscle relaxant activity via chelating the calcium ions and by blocking the calcium channels.

### REFERENCES

1. Dam H: The antihemorrhagic vitamin of the chick: occurrence and chemical nature. Nature 1935, 135:652-653.
2. Berkner KL, Pudota BN: Vitamin K-dependent carboxylation of the carboxylase. Proc Natl Acad Sci U S A 1998, 95(2):466-471.
3. Stenflo J: A new vitamin K-dependent protein. Purification from bovine plasma and preliminary characterization. J Biol Chem 1976, 251(2):355-363.
4. Lian JB, Hauschka PV, Gallop PM: Properties and biosynthesis of a vitamin K-dependent calcium binding protein in bone. Fed Proc 1978, 37(12):2615-2620.
5. Thomson RH: Naturally Occurring Quinones. New York, N.Y.: Academic Press; 1971.
6. Davidson RT, Foley AL, Engelke JA, Suttie JW: Conversion of dietary phylloquinone to tissue menaquinone-4 in rats is not dependent on gut bacteria. J Nutr 1998, 128(2):220-223.
7. Thijssen HH, Drittij-Reijnders MJ: Vitamin K status in human tissues: tissue-specific accumulation of phylloquinone and menaquinone-4. Br J Nutr 1996, 75(1):121-127.
8. Seegers WH, Bang NU: Blood Clotting Enzymology. New York, NY: Academic Press; 1967.
9. Billeter M, Bolliger W, Martius C: Untersuchungen uber die umwandlung von verfutterten K-vitamin durch austausch der seitenkette und die rolle der darmbakterien hierbei. Biochem Z 1964(340):290-303.
10. Furie B, Furie BC: Molecular and cellular biology of blood coagulation. N Engl J Med 1992, 326(12):800-806.
11. Booth SL, Suttie JW: Dietary intake and adequacy of vitamin K. J Nutr 1998, 128(5):785-788.
12. Gijsbers BL, Jie KS, Vermeer C: Effect of food composition on vitamin K absorption in human volunteers. Br J Nutr 1996, 76(2):223-229.
13. Schurgers LJ, Vermeer C: Determination of phylloquinone and menaquinones in food. Effect of food matrix on circulating vitamin K concentrations. Haemostasis 2000, 30(6):298-307.
14. Ushiroyama T, Ikeda A, Ueki M: Effect of continuous combined therapy with vitamin K(2) and vitamin D(3) on bone mineral density and coagulofibrinolysis function in postmenopausal women. Maturitas 2002, 41(3):211-221.
15. Ronden JE, Thijssen HH, Vermeer C: Tissue distribution of K-vitamers under different nutritional regimens in the rat. Biochim Biophys Acta 1998, 1379(1):16-22.
16. Thijssen HH, Drittij Reijnders MJ: Vitamin K distribution in rat tissues: dietary phylloquinone is a source of tissue menaquinone-4. Br-J-Nutr 1994, 72(3):415-425 issn: 0007-1145.
17. Will BH, Usui Y, Suttie JW: Comparative metabolism and requirement of vitamin K in chicks and rats. J-Nutr 1992, 122(12):2354-2360 issn: 0022-3166.
18. Thijssen HH, Drittij Reijnders MJ, Fischer MA: Phylloquinone and menaquinone-4 distribution in rats: synthesis rather than uptake determines menaquinone-4 organ concentrations. J Nutr 1996, 126(2):537-543.
19. Ronden JE, Drittij-Reijnders MJ, Vermeer C, Thijssen HHW: Intestinal flora is not an intermediate in the phylloquinone- menaquinone-4 conversion in the rat. Biochim Biophys Acta 1998, 1379(1):69-75.
20. Okano T, Shimomura Y, Yamane M, Suhara Y, Kamao M, Sugiura M, Nakagawa K: Conversion of phylloquinone (Vitamin K1) into menaquinone-4 (Vitamin K2) in mice: two possible routes for menaquinone-4 accumulation in cerebra of mice. J Biol Chem 2008, 283(17):11270-11279.
21. Cockayne S, Adamson J, Lanham-New S, Shearer MJ, Gilbody S, Torgerson DJ: Vitamin K and the prevention of fractures: systematic review and meta-analysis of randomized controlled trials. Arch Intern Med 2006, 166(12):1256-1261.
22. Geleijnse JM, Vermeer C, Grobbee DE, Schurgers LJ, Knapen MH, van der Meer IM, Hofman A, Witteman JC: Dietary intake of menaquinone is associated with a reduced risk of coronary heart disease: the Rotterdam Study. J Nutr 2004, 134(11):3100-3105.
23. Kamao M, Suhara Y, Tsugawa N, Uwano M, Yamaguchi N, Uenishi K, Ishida H, Sasaki S, Okano T: Vitamin K content of foods and dietary vitamin K intake in Japanese young women. J Nutr Sci Vitaminol (Tokyo) 2007, 53(6):464-470.
24. Kaneki M, Hedges SJ, Hosoi T, Fujiwara S, Lyons A, Crean SJ, Ishida N, Nakagawa M, Takechi M, Sano Y et al: Japanese fermented soybean food as the major determinant of the large geographic difference in circulating levels of vitamin K2: possible implications for hip-fracture risk. Nutrition 2001, 17(4):315-321.
25. Tsukamoto Y, Ichise H, Yamaguchi M: Prolonged Intake of Dietary Fermented Soybeans (Natto) with the Reinforced Vitamin K2 (Menaquinone-7) Enhances Circulating γ - Carboxylated Osteocalcin Concentration in Normal Individuals. Journal of Health Science 2000, 46(4):317-321.
26. Berkner KL, Runge KW: The physiology of vitamin K nutriture and vitamin K-dependent protein function in atherosclerosis. J Thromb Haemost 2004, 2(12):2118-2132.
27. Evans CJ, Fowkes FG, Ruckley CV, Lee AJ: Prevalence of varicose veins and chronic venous insufficiency in men and women in the general population: Edinburgh Vein Study. J Epidemiol Community Health 1999, 53(3):149-153.
28. Bergan JJ, Schmid-Schonbein GW, Smith PD, Nicolaides AN, Boisseau MR, Eklof B: Chronic venous disease. N Engl J Med 2006, 355(5):488-498.
29. White JV, Ryjewski C: Chronic venous insufficiency. Perspect Vasc Surg Endovasc Ther 2005, 17(4):319-327.
30. Roffe C, Sills S, Crome P, Jones P: Randomised, cross-over, placebo controlled trial of magnesium citrate in the treatment of chronic persistent leg cramps. Med Sci Monit 2002, 8(5):CR326-330.
31. Naylor JR, Young JB: A general population survey of rest cramps. Age Ageing 1994, 23(5):418-420.
32. Abdulla AJ, Jones PW, Pearce VR: Leg cramps in the elderly: prevalence, drug and disease associations. Int J Clin Pract 1999, 53(7):494-496.
33. Raper HSPR, 245-282: PhysiolRev 1928, 8:245-282.
34. Mason HS: J Biol Chem 1948, 172:83-89.
35. Lavallee CR, Chalifoux JR, Moosally AJ, Balkema GW: Elevated free calcium levels in the subretinal space elevate the absolute dark-adapted threshold in hypopigmented mice. J Neurophysiol 2003, 90(6):3654-3662.
36. de Boer-van den Berg MA, Verstijnen CP, Vermeer C: Vitamin K-dependent carboxylase in skin. J Invest Dermatol 1986, 87(3):377-380.
37. Shah NS, Lazarus MC, Bugdodel R, Hsia SL, He J, Duncan R, Baumann L: The effects of topical vitamin K on bruising after laser treatment. Journal of the American Academy of Dermatology 2002, 47(2):241-244.
38. Kovacs RK, Bodai L, Dobozy A, Kemeny L: Lack of the effect of topical vitamin K on bruising after mechanical injury. Journal of the American Academy of Dermatology 2004, 50(6):982-983.
39. Prandoni P, Lensing AW, Cogo A, Cuppini S, Villalta S, Carta M, Cattelan AM, Polistena P, Bernardi E, Prins MH: The long-term clinical course of acute deep venous thrombosis. Ann Intern Med 1996, 125(1): 1-7.
40. Kahn SR, Ginsberg JS: Relationship between deep venous thrombosis and the postthrombotic syndrome. Arch Intern Med 2004, 164(1):17-26.
41. Stain M, Schonauer V, Minar E, Bialonczyk C, Hirschl M, Weltermann A, Kyrle PA, Eichinger S: The post-thrombotic syndrome: risk factors and impact on the course of thrombotic disease. J Thromb Haemost 2005, 3(12):2671-2676.
42. Schulman S, Lindmarker P, Holmstrom M, Larfars G, Carlsson A, Nicol P, Svensson E, Ljungberg B, Viering S, Nordlander S et al: Post-thrombotic syndrome, recurrence, and death 10 years after the first episode of venous thromboembolism treated with warfarin for 6 weeks or 6 months. J Thromb Haemost 2006, 4(4):734-742.
43. Kurz X, Kahn SR, Abenhaim L, Clement D, Norgren L, Baccaglini U, Berard A, Cooke JP, Cornu-Thenard A, Depairon M et al: Chronic venous disorders of the leg: epidemiology, outcomes, diagnosis and management. Summary of an evidence-based report of the VEINES task force. Venous Insufficiency Epidemiologic and Economic Studies. Int Angiol 1999, 18(2):83-102.

## Claims

1. A composition for oral administration comprising a therapeutically effective amount of vitamin K2 either alone or in combination with one or more therapeutically effective active agents for use in the improvement of blood perfusion and the amelioration of hypoxia in venous insufficiency and its manifestations in human subjects, wherein the venous insufficiency is not varicose veins,
for use in the relief from the signs and symptoms of chronic venous insufficiencies in human subjects, as a phlebodynamic agent; wherein the venous insufficiency manifestations are selected from the group consisting of paresthesiae, itching, cramps and heaviness of limbs; or
for use in the treatment of post thrombotic syndrome (PTS) complications in human subjects, wherein the PTS complications are selected from the group consisting of heaviness, cramps, itching and tingling in limbs.

2. The composition according to Claim 1, wherein the oral administration comprises a therapeutically effective amount of vitamin K2-7.

## Patentansprüche

1. Zusammensetzung zur oralen Verabreichung, die eine therapeutisch wirksame Menge von Vitamin K2, entweder allein oder in Kombination mit einem oder mehreren therapeutisch wirksamen Wirkstoffen, umfasst, für die Verwendung bei der Verbesserung von Durchblutung und der Besserung von Hypoxie bei venöser Insuffizienz und ihren Manifestationen bei menschlichen Subjekten, wobei die venöse Insuffizienz nicht Krampfadern ist,
für die Verwendung bei der Linderung der Anzeichen und Symptome von chronisch venösen Insuffizienzen bei menschlichen Subjekten als phlebodynamisches Mittel; wobei die Manifestationen von venöser Insuffizienz aus der Gruppe ausgewählt sind, die aus Parästhesien, Juckreiz, Krämpfen und Schweregefühl in den Gliedmaßen besteht; oder
für die Verwendung bei der Behandlung von Komplikationen mit postthrombotischem Syndrom (PTS) bei menschlichen Subjekten, wobei die PTS-Komplikationen aus der Gruppe ausgewählt sind, die aus Schweregefühl, Krämpfen, Juckreiz und Kribbeln in den Gliedmaßen besteht.

2. Zusammensetzung nach Anspruch 1, wobei die orale Verabreichung eine therapeutisch wirksame Menge von Vitamin K2-7 umfasst.

## Revendications

1. Composition destinée à une administration orale, comprenant une quantité thérapeutiquement efficace de vitamine K2 soit seule, soit en combinaison avec un ou plusieurs agents actifs thérapeutiquement efficaces,
pour une utilisation dans l'amélioration de la perfusion sanguine et l'amélioration de l'hypoxie dans l'insuffisance veineuse et ses manifestations chez des sujets humains, où l'insuffisance veineuse n'est pas constituée par les varices ;
pour une utilisation dans le soulagement des signes et des symptômes d'insuffisances veineuses chroniques chez des sujets humains, comme agent phlébo-dynamique ; où les manifestations d'insuffisance veineuse sont choisies dans le groupe constitué par la paresthésie, le prurit, les crampes et la lourdeur des membres ; ou
pour une utilisation dans le traitement des complications du syndrome post-thrombotique (PTS) chez des sujets humains, où les complications du PTS sont choisies dans le groupe constitué par la lourdeur, les crampes, le prurit et les fourmillements des membres.

2. Composition selon la revendication 1, dans laquelle l'administration orale comprend une quantité thérapeutiquement efficace de vitamine K2-7.
